# EUROPEAN PATENT SPECIFICATION

(11) **EP 1 192 175 B1**
(45) Date of publication and mention of the grant of the patent: **05.03.2003**
(21) Application number: 00944084.3
(22) Date of filing: 04.07.2000
(51) Int. Cl.: C07J 9/00, B01D 11/02

(54) **METHOD OF TREATING ORGANIC SUBSTANCES**
VERFAHREN ZUR BEHANDLUNG ORGANISCHER SUBSTANZEN
METHODE DE TRAITEMENT DE SUBSTANCES ORGANIQUES

(30) Priority: 05.07.1999 FI 991533
(43) Date of publication of application: 03.04.2002
(73) Proprietor: Danisco Sugar Oy, 02150 Espoo (FI)
(72) Inventor: AALTONEN, Olli, FIN-00330 Helsinki (FI); HAARASILTA, Sampsa, FIN-04230 Kerava (FI); PIIROINEN, Pekka, FIN-02400 Kirkkonummi (FI); RANTAKYLÄ, Markku, FIN-00400 Helsinki (FI); VIRTANEN, Jouko, FIN-15560 Nastola (FI); KAIPAINEN, Antti, FIN-02130 Espoo (FI)
(74) Representative: Puranen, Maija-Liisa
(86) International application number: FI0000612
(87) International publication number: WO01002421

(56) References cited:
- EP-A- 0 541 853
- WO-A-98/06405
- GB-A- 2 343 452
- US-A- 3 879 431
- US-A- 5 770 749
- PATENT ABSTRACTS OF JAPAN vol. 012, no. 028 (C-471), 27 January 1988 (1988-01-27) & JP 62 178596 A (SHOWA SANGYO KK), 5 August 1987 (1987-08-05)

## Description

### BACKGROUND OF THE INVENTION

The invention relates to a method of purifying phytosterol concentrates by treating the sterol concentrate with a pressurized fluid, typically with carbon dioxide. With the method of the invention, it is possible to remove from the sterol concentrates undesirable odours and flavours that hamper their use as foodstuffs as well as other harmful components and possible solvent residues. The invention also relates to a phytosterol concentrate obtained by the method and a foodstuff premix or an animal feed premix containing the same and a foodstuff or an animal feed containing said premixes. Furthermore, the invention relates to a phytosterol concentrate which is substantially free of substances causing undesirable odours and flavours and of other harmful components.

Phytosterols or plant sterols refer to naturally occurring compounds having a steroid backbone. The most common, naturally occurring phytosterols include β-sitosterol, campesterol, stigmasterol and brassicasterol.

The cholesterol-lowering effect of phytosterols has been known since the 1950s and several reports on the positive effects of the plant sterols on the cholesterol level are found in the literature of the field. The most recent reports include, for instance, that by Heineman et al., Eur. J. Clin. Pharmacol. 40 (Suppl.1), 1991, pp. 59-63, on the effect of sitostanol and sitosterol on the cholesterol absorption in the intestines by using an intestinal perfusion technique. Sitostanol was found to reduce cholesterol absorption by as much as 85 %, sitosterol by 50 %.

Phytosterol concentrates are manufactured industrially by various extraction and crystallization methods in which a distillate obtained from steam refining of vegetable oils and a crude soap obtained from pulp cooking are typically used as raw materials.

As regards undesirable odours and flavours and other harmful components, vegetable-oil-based, such as soybean-based, phytosterol concentrates are particularly problematic. In preparation, these concentrates retain some unsaturated fatty acids whose rancidification products cause unpleasant odours and flavours which hamper the use in foodstuffs. Typical rancidification products include, for instance, hexanal and methyl ester of octanoic acid. It has been problematic to remove impurities producing undesirable odours and flavours from the phytosterol concentrates, because the impurities tend to migrate in separation processes with the sterols.

In conventional purification methods based on extracting and crystallizing sterols, solvent residues tend to remain to some extent in the sterol concentrates, which residues are also harmful to the use of sterol concentrates as foodstuffs.

For use in pharmaceuticals and foodstuffs, a crude phytosterol concentrate has been typically purified by crystallization. For instance, US patent 2,843,610 (Eastman Kodak Co.) and US patent 4,420,427 (Kaukas Oy) disclose a process for crystallizing β-sitosterol from methanol/ketone/water solutions by cooling. By repeating the recrystallization procedures several times, high phytosterol concentration is achieved, which is often sufficient for pharmaceutical use. However, minor amounts of oxygen-containing compounds, which produce odour and flavour that hamper the use in foodstuffs, may remain in the crystallized phytosterol products.

US patent 4,153,622 (Medipolar) discloses a process for the isolation of β-sitosterols from sterol mixtures obtained from a neutral fraction of a pulp cooking process. In this process an acetone solution containing β-sitosterol is filtered with active carbon to remove impurities, after which ethanol is added to the obtained solution and β-sitosterol is crystallized from the acetone/ethanol solution by cooling. In crystallizations and filtrations with active carbon, it is necessary to use multiple amounts of flammable and partly toxic solvents in comparison with the amount of phytosterol. After crystallizations and filtrations the solvents must be evaporated from the phytosterol product. Despite the evaporation, at least small amounts of solvents will remain in the phytosterol.

Extraction with supercritical fluids has been used for extracting sterols from plant material. One method of this kind is set forth in US patent 5,252,729 (Schering Co.). In this method, the sterols are extracted from an acid-hydrolysate of plant material with a supercritical fluid, such as carbon dioxide, optionally with an organic co-solvent. The desired sterols dissolve in the supercritical fluid, from which they are recovered. The temperature used in extraction is preferably 75 to 250 °C and the pressure is 100 to 300 atm (about 101 to 304 bar). In this method, impurities causing undesirable odours and flavours cannot be separated from the sterols, since the impurities dissolve in the carbon dioxide together with the sterols. The sterol concentrate thus contains both sterols and impurities.

From EP application 0 541 853 A1 (Müller-Extract-Company) is known the use of a supercritical fluid for improving the preservability of palm oil extract and the stability of β-sitosterol contained therein and for removing unpleasant odour and flavour agents contained in the palm oil extract. β-sitosterol content in the palm oil extract is very small, about 1 %. In this method, the palm oil extract is treated with pressurized, liquid carbon dioxide. The treatment is carried out in two steps, one treatment under a pressure of 75 to 350 bar and at a temperature of 20 to 90 °C and the other at a temperature of 15 to 30 °C and under a pressure of 40 to 75 bar. The described method yields a deodorized palm oil extract which has a very small β-sitosterol content (about 1 %).

However, the use of pressurized fluids for purifying the phytosterol concentrates has not been reported in the literature.

### DEFINITIONS ASSOCIATED WITH THE INVENTION

In connection with the present invention, phytosterols refer to free sterols and stanols and derivatives thereof, such as esters and glycosides. In the present invention, typical phytosterols include β-sitosterol, campesterol, stigmasterol and brassicasterol.

Phytosterol concentrates refer to a phytosterol preparation which contains above-defined phytosterols and whose phytosterol content is raised. The phytosterol concentrate can be a phytosterol preparation of natural origin. The phytosterol concentrate can be based on tall oil, for instance, typically originating from a soap fraction of a pulp cooking process. The phytosterol concentrate may also originate from separation processes of phytogenic tocopherols, for instance from a distillation fraction obtained from steam refining of vegetable oils. The vegetable oil raw material is typically soybean-, rapeseed-, sunflower- or maize-based vegetable oil.

The phytosterol concentrate can be in a solid form, whereby its phytosterol content is typically over 80 %, preferably 90 to 98 %. The phytosterol concentrate can also be in the form of a liquid or suspension, whereby its phytosterol content is typically in the range of 20 to 80 %, preferably 40 to 60 %.

In connection with the present invention, the pressurized fluid refers to a food-grade, non-polar substance or mixture of substances that is gaseous at normal atmospheric pressure and that is used in a pressurized state in the present invention. The density of the pressurized fluid should be sufficient to dissolve the impurities of the sterol concentrate. To achieve sufficient dissolving properties, the minimum pressure of the fluid is typically 50 bar.

Preferably, the pressurized fluid to be used according to the invention mainly consists of carbon dioxide. In addition to carbon dioxide, it is also possible to use propane, for instance. The pressurized fluid can also contain solvents, such as alcohols with low molecular weight, for instance ethanol.

### BRIEF DESCRIPTION OF THE INVENTION

According to the present invention, it has been found that by treating a phytosterol concentrate with a pressurized fluid, typically with carbon dioxide, it is possible to remove components producing undesirable odours and flavours selectively without losing sterols with the fluid. The method enables the preparation of a solid, food-grade, pure phytosterol product without multistage crystallization and without flammable or toxic organic solvents.

Separation of carbon dioxide from a purified phytosterol concentrate does not require any special measures. When the method is performed on a continuous or semi-continuous basis, the carbon dioxide stream containing impurities is simply diverted from the purified phytosterol concentrate. It is also possible to allow the carbon dioxide to evaporate from the purified phytosterol.

### DETAILED DESCRIPTION OF THE INVENTION

The object of the invention is to provide a method of purifying phytosterol concentrates, in which method the phytosterol concentrate is contacted with a pressurized fluid. The temperature and pressure to be used are typically in the range of -20...180 °C and 50 to 600 bar.

The pressurized fluid is typically a food-grade, non-polar substance or mixture of substances that is gaseous at normal atmospheric pressure. The fluid to be used may also contain solvents, such as alcohols with low molecular weight, e.g. ethanol.

It is advantageous to use a pressurized fluid which mainly consists of carbon dioxide. Carbon dioxide is an environment-friendly, pure gas which does not provide additional impurities in the final product to be purified. However, it is also possible to use other food-grade gases, such as propane.

The method is suitably performed at a temperature of 10 to 75 °C, preferably at 20 to 60 °C, most preferably at 35 to 45 °C. A suitable pressure range is 80 to 300 bar, preferably 150 to 250 bar.

Technically, the method works within the entire above-mentioned temperature and pressure range of -20...180 °C and 50 to 600 bar. The higher the working temperature, the more sterols start dissolving in the carbon dioxide, whereby the yield of sterols decreases while the purity of the phytosterol concentrate increases. At lower temperatures, the sterol losses are lower, but correspondingly, the product may contain more impurities. The lower the pressure, the lower the density of gas and the poorer its dissolving power.

The phytosterol concentrate used can be a tall-oil-based phytosterol concentrate, whereby it typically originates from a soap fraction of a pulp cooking process. It may also originate from separation processes of phytogenic tocopherols, for instance, from a distillation fraction from steam refining of vegetable oils. The raw material that is based on vegetable oil is typically soybean-, rapeseed-, sunflower or maize-based vegetable oil.

The phytosterol concentrate can be in a solid form, whereby its phytosterol content is typically over 80 %, preferably 90 to 98 %. The phytosterol concentrate can also be in the form of a liquid or suspension, whereby its phytosterol content is typically within the range of 20 to 80 %, preferably 40 to 60 %.

In the following particular embodiments of the invention, the method of the invention is described as applied to a carbon dioxide treatment.

The method is preferably performed as a semi-continuous process, whereby the phytosterol concentrate to be treated is placed in a pressure vessel and it is contacted with a continuous, pressurized carbon dioxide stream. Substances producing undesirable odours and flavours, colouring agents and possible solvent residues are released from the phytosterol concentrate with the carbon dioxide. Thereafter, the carbon dioxide stream that has been in contact with the phytosterol concentrate and that contains impurities is diverted on a continuous basis from the phytosterol concentrate. The purified phytosterol concentrate is recovered. A semi-continuous process is particularly well suited for phytosterol concentrates in solid form.

The method can also be implemented as a batch process or as a continuous process.

In one embodiment of the invention, the carbon dioxide treatment can be combined with other purification methods of phytosterols, such as crystallization and extraction methods. In that case, raw material is suitably a phytosterol concentrate, the phytosterol content of which is still low, for instance, about 5 to 50 %. The carbon dioxide treatment can be carried out prior to the crystallization/extraction steps and/or thereafter. The carbon dioxide treatment removes impurities that inhibit crystallization (crystallization inhibitors), which facilitates crystallization steps to be carried out later and the yield from crystallization improves. When the carbon dioxide treatment is carried out after the crystallization/extraction steps, solvent residues originating from the extraction solvents can be removed.

In the extraction step, typical extraction solvents include hydrocarbons, such as hexane, and ketones, e.g. acetone. Typical crystallization solvents are aqueous alcohol solutions which may also contain ketones.

The carbon dioxide treatment in accordance with the invention can thus replace, completely or partly, purification steps implemented with organic solvents, and consequently, production costs can be reduced, use of organic solvents can be reduced and a higher yield of phytosterols can be achieved.

In a further embodiment of the method of the invention, the carbon dioxide treatment of the invention can be combined with the preparation of the actual phytosterol concentrate. In that case, the phytosterols are separated from the raw material used with the same fluid that is used for separating impurities. The conditions (pressure and temperature) are selected such that first is separated a phytosterol-rich concentrate with impurities, and thereafter the impurities are separated from the phytosterol concentrate. In that case, a phytosterol preparation dissolved in a supercritical fluid is used as the starting material in the method of the invention.

The last-mentioned embodiment of the invention comprises a preliminary stage in which a crude phytosterol preparation is first extracted with supercritical carbon dioxide (the pressure and temperature of the carbon dioxide both being simultaneously above the critical point of carbon dioxide) at a high pressure and at an elevated temperature. Thus, most of the phytosterol concentrate and a portion of the impurities dissolve in the carbon dioxide stream. The portion of the impurities that is insoluble in carbon dioxide can be separated in a supercritical extraction step. The carbon dioxide stream that contains as dissolved most of the phytosterols and impurities soluble in carbon dioxide is conducted through a reduction in pressure and temperature to a pressure and temperature range used in the method of the invention. The phytosterols then precipitate in the pressure vessel and the substances producing undesirable odours and flavours remain dissolved in the carbon dioxide stream, which is diverted from the purified, precipitated phytosterol concentrate.

The invention also relates to a purified phytosterol concentrate which is obtained by the method of the invention.

The invention further relates to a solid phytosterol concentrate, the phytosterol content of which is over 80 %, preferably 90 to 98 %, and which is substantially free of substances producing undesirable odours and flavours and of other harmful components.

The invention also relates to a phytosterol concentrate in the form of a liquid or suspension, the phytosterol content of which is 20 to 80 %, preferably 40 to 60 %, and which is substantially free of substances producing undesirable odours and flavours and of other harmful components.

The invention further relates to a foodstuff premix containing phytosterol concentrate of the invention and conventional foodstuff raw material. Said conventional foodstuff raw material is typically selected from the group comprising cereal, leguminous plants, milk powder and/or fruits, vegetables and berries, particularly in powder form, as well as bone, feather, fish, meat and/or rind. The raw material can also be e.g. fat or a fat mixture, sugar or a sugar mixture, a special sweetener or salt or a salt mixture.

In connection with the present invention, foodstuffs also comprise so-called dietary supplement products and products classifiable as pharmaceuticals.

The premix of the invention is typically a so called premix whereto considerable amounts of other substances are added in connection with the use. The premix can also be a so called complete mix that is not supplemented with other substances in connection with the use, possibly apart from water or other liquid.

One example of the premixes of the invention is a mineral-salt-based premix which contains the phytosterol concentrate of the invention and a mineral/minerals which is/are selected from the group of magnesium, calcium and potassium. The premix is also useful as an animal feed premix.

Another example of the premixes of the invention is a premix which contains the phytosterol concentrate of the invention and an emulsifier and optionally also a fat component. The premix can be in solid form or in the form of an oil or suspension. The premix is also useful as an animal feed premix.

The premix of the invention can also be a premix which is intended for sweets products and contains the phytosterol concentrate of the invention and conventional sweets raw material. The premix may also be a chocolate premix containing the phytosterol concentrate of the invention and conventional chocolate raw material. One example is a sugar-based premix containing the phytosterol concentrate of the invention and saccharose. The amount of the phytosterol is typically 5 to 25 %, preferably 10 to 20 %.

One more example of the premixes of the invention is a premix for sausage products which contains the phytosterol concentrate of the invention and conventional sausage raw material.

The premix of the invention may also be a premix intended for bakery products which contains the phytosterol preparation of the invention and conventional baking raw material. This premix is typically a cereal-based premix. One premix of this kind is a premix containing the phytosterol concentrate and flour. The flour is typically rye, buckwheat, maize or wheat flour and the premix typically contains about 5 to 25 %, more preferably about 10 to 20 % of phytosterol.

The premix of the invention may also be a premix intended for milk products which contains the phytosterol concentrate of the invention and conventional milk product raw material. One example is a premix intended for yoghurt products.

The amount of the purified phytosterol concentrate in the premix may vary widely depending on the composition and field of use of the premix. In general, the amount of the phytosterol is about 1 to 60 % by weight, preferably about 5 to 50, more preferably about 10 to 40 and most preferably about 10 to 20 % by weight.

The premix is typically prepared such that a homogenous, finely divided mixture is formed from the phytosterol concentrate and a conventional foodstuff raw material that is defined above.

The invention also relates to foodstuffs which contain the phytosterol concentrate of the invention or the foodstuff premix of the invention.

The invention further relates to premixes intended for animal feeds and containing the phytosterol preparation of the invention and conventional animal feed raw material. Additionally, the invention relates to an animal feed containing the phytosterol preparation of the invention or the animal feed premix of the invention.

The following examples describe in greater detail some embodiments of the invention. However, the invention is not restricted to the implementations described in the examples.

### Example 1

118.1 g of soybean-based, powdery phytosterol concentrate was introduced in a tubular pressure vessel (capacity 200 ml), the upper and lower end of which was provided with a porous metal sinter. The phytosterol content of the concentrate was 96 % (determined by gas chromatogrgaphy) and its phytosterol composition was as follows: 28 % campesterol, 42 % β-sitosterol and 26 % stigmasterol. The phytosterol powder was placed in a space between the sinters at the upper and lower ends of the pressure vessel.

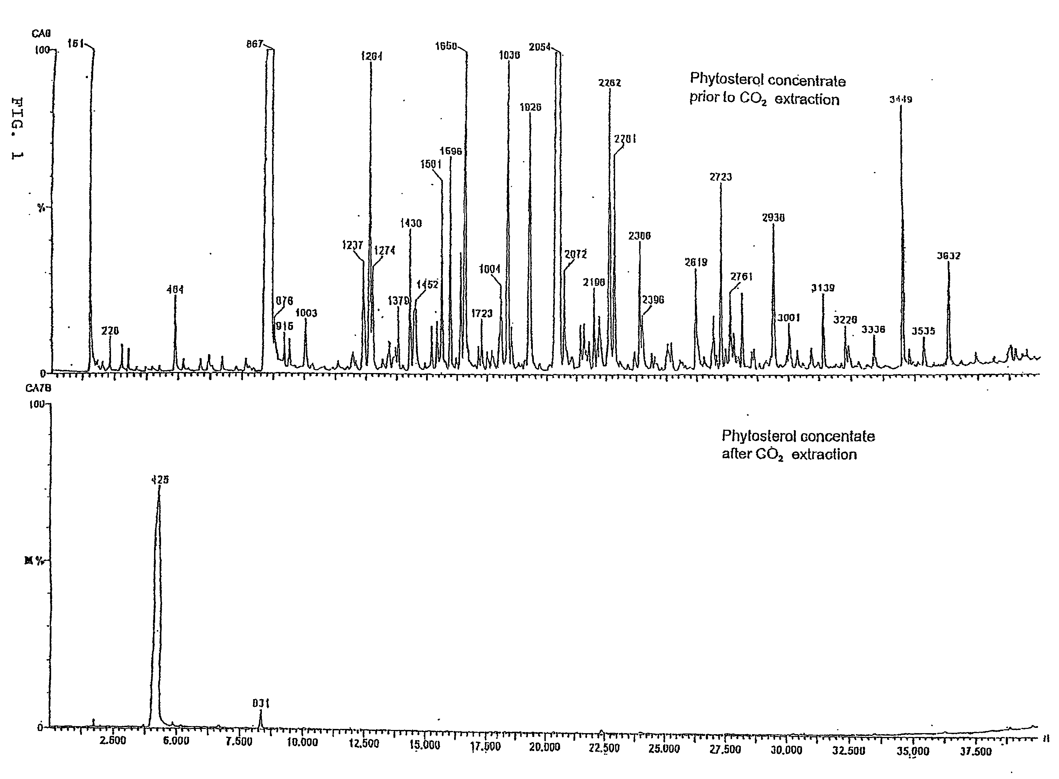

The phytosterol powder used as a starting material was yellowish grey in colour and it had a strong odour. A gas chromatographic head-space analysis showed that the powder contained tens of volatile components. Quantitatively, the main components were hexanal and methyl ester of octanoic acid (see the upper spectrum in Figure 1, the hexanal peak at point 867 and the peak of the methyl ester of octanoic acid at point 2054).

The pressure vessel containing impure phytosterol powder was heated up to 40 °C, and carbon dioxide was continuously conducted through the vessel at a pressure of 235 to 240 bar, at a rate of 6 g/min. The carbon dioxide stream that discharges from the pressure vessel was conducted through a pressure reducing valve to a glass vessel having normal atmospheric pressure, whereby the carbon dioxide discharged as gas and brownish liquid started accumulating in the vessel. During the first 50 minutes, 0.57 g of liquid was accumulated. When liquid accumulation stopped, the carbon dioxide was stopped to pass through the sterol powder. Altogether, the amount of brownish liquid extracted from the sterol powder in this manner was 1 % of the weight of the phytosterol powder. All the compounds detected in the head-space analysis of the non-purified sterol powder were recognizable in a chromatographic analysis of the extract. Also the main components were the same.

It appears from Figure 1 that the carbon dioxide treatment had succeeded in removing the harmful components from the sterol powder (see the lower spectrum in Figure 1). As regards the odour, the purified sterol powder emitted only an extremely weak, unidentifiable odour. The purified sterol powder was white in colour.

### Example 2

The same test arrangement was used as in Example 1.

96.9 g of the same soybean-based phytosterol concentrate as above was weighted in the space between the sinters of the pressure vessel, and liquid carbon dioxide was pumped through the pressure vessel at a temperature of 25 °C and at a pressure of 150 bar. The extract obtained was first yellowish, semisolid matter with a strong odour. When the extract had turned into a white, solid matter, the carbon dioxide was stopped to pass through the sterol powder. In this manner, 0.84 g of extract was obtained from the phytosterol powder. The phytosterol concentrate extracted with the carbon dioxide was nearly white in colour and it emitted a weak, unidentifiable odour.

### Example 3

An impure, soybean-oil-based phytosterol concentrate was prepared first. A steam distillate, which was obtained from deodorizing step of soybean oil and which contains about 5 % of phytosterols, was saponified and acidified as described in Example 1 of US patent 2,843,610. The obtained oil fraction was dissolved in a boiling mixture of acetone, methanol and water, and the solution was allowed to cool to room temperature. A precipitate was crystallized from the solution and the precipitate was filtered from the mother liquor.

The obtained, solvent-containing phytosterol concentrate in the form of a yellowish precipitate is extracted by using the test arrangement described in Example 1. The extraction is performed with carbon dioxide, at a temperature of 50 °C and at a pressure of 180 bar. The product obtained is nearly white, odourless and solid phytosterol with a phytosterol concentration exceeding 96 %. No significant acetone or methanol residues, nor volatile components, are detected in a gas chromatographic head-space analysis of the product.

### Example 4

The same test arrangement is used as in Example 1. Impure phytosterol preparation is introduced in the pressure vessel, and carbon dioxide is passed through the pressure vessel at a temperature of 150 °C and at a pressure of 380 bar at most. In these conditions, most of the phytosterols and the impurities soluble in carbon dioxide dissolve in the carbon dioxide stream.

The carbon dioxide stream, which comes from the pressure vessel and which contains most of the phytosterols and impurities soluble in carbon dioxide, is passed on a continuous basis through a pressure reducing valve to a second pressure vessel having a temperature of 40 °C and a pressure of 240 bar. Thus, the phytosterols precipitate, while the carbon-dioxide-soluble impurities remain dissolved in the carbon dioxide stream. From this pressure vessel the carbon dioxide stream is still continuously forwarded through the next pressure reducing valve to a vessel having a normal atmospheric pressure. After the test, a small amount of yellowish matter, which remained insoluble in carbon dioxide (impurities insoluble in carbon dioxide), is collected from the first pressure vessel. From the second pressure vessel is collected pure white, odourless, phytosterol concentrate (a precipitated phytosterol product). Brownish, oily matter (impurities soluble in carbon dioxide) accumulate in a glass vessel having a normal atmospheric pressure.

### Example 5

### Examples of foodstuff premixes

### (A) Cereal-based premix

2 kg of phytosterol preparation, which is obtained according to Example 1 and which is pulverized by impact milling or jet pulverizing, is carefully mixed with 18 kg of grains. The grains can be rye grains or wheat grains husked in various ways. The mixture is ground with a grinder based on the impact milling principle and represented by the Atrex mill (Megatrex Oy, Harjavalta).

Processing conditions were as follows:

| | |
|---|---|
| Upper rotor (rpm) | 3000 |
| Lower rotor (rpm) | 3000 |
| Mass flow of grains (kg/min) | 2.5 |
| Temperature of rye (°C) | 25 |

When passing through the grinder, the grains are crushed and the phytosterol particles are ground with the grains into a homogenous phytosterol-flour mixture.

A cereal-based, phytosteroi-containing foodstuff premix is obtained, which is useful for preparing bakery products, for instance.

### (B) Sugar-based premix

20 g of phytosterol preparation obtained according to Example 1 and pulverized by jet pulverizing or impact milling is mixed with 180 kg of saccharose and the mixture is jet-pulverized with a dry collision grinder using a classifier (Oy Finnpulva Ab, grinder FP3P, classifier FPC15R). The carrier gas was air having a temperature of 63 °C and a feed pressure of 2.4 bar.

A sugar-based, phytosterol-containing foodstuff premix is obtained, which is useful for preparing chocolate, for instance.

### (C) Sausage containing phytosterol concentrate

A sausage was prepared by using a conventional cooked sausage recipe with following ingredients:

| | |
|---|---|
| Beef-based ingredients | 30 - 40 % |
| Pork-based ingredients | 30 % |
| Water | 25 % |
| Milk powder and/or potato flour, wheat flour | 5 % |
| Salt | 1.5 % |
| Admixtures (phosphates, etc) | 1 - 2 % |

Phytosterol was added by replacing milk powder with a mixture of milk powder and phytosterol in which mixture the proportion of phytosterol was 20 %. A product of a Finnish ring sausage type was prepared of the mixture.

### Example 6

### Preparation of an animal feed containing phytosterol concentrate

A chicken feed was prepared by using the following basic recipe of chicken feed:

| | |
|---|---|
| Starch raw material (mainly barley) | 60 - 70 % |
| Protein component (mainly crushed soybean) | 20 - 30 % |
| Other ingredients | 5 - 10 % |
| - minerals (mainly CaCo₃) | |
| - vitamins | |
| - trace elements | |
| - amino acids | |
| - enzymes, if any | |

The phytosterol concentrate of the invention was ground with CaCO₃ into a premix containing about 20 % of phytosterol. This premix was added to other animal feed components so that the final phytosterol content of the animal feed mixture was 1 %.

It is obvious to the person skilled in the art that as technology progresses the basic idea of the invention can be implemented in a variety of ways. Thus, the invention and its embodiments are not restricted to the above-described examples, but they may vary within the scope of the claims.

## Claims

1. A method of purifying phytosterol concentrates, **characterized in that** the phytosterol concentrate having a phytosterol content of 20 to 80 % or over 80 % is contacted with a pressurized fluid.

2. A method as claimed in claim 1, **characterized in that** the pressurized fluid is a food-grade, non-polar substance or mixture of substances that is gaseous at normal atmospheric pressure.

3. A method as claimed in claim 2, **characterized in that** the pressurized fluid mainly consists of carbon dioxide.

4. A method as claimed in any one of claims 1 to 3, **characterized in that** the method is carried out at a temperature of -20 °C ... 180 °C and at a pressure of 50 to 600 bar.

5. A method as claimed in claim 4, **characterized in that** the method is carried out at a temperature of 10 to 75 °C, preferably at 20 to 60 °C, most preferably at 35 to 45 °C and at a pressure of 80 to 300 bar, preferably at 150 to 250 bar.

6. A method as claimed in any one of claims 1 to 5, **characterized in that** the phytosterol concentrate is obtained from a distillation fraction of steam refining of vegetable oils.

7. A method as claimed in any one of claims 1 to 5, **characterized in that** the phytosterol concentrate is obtained from a soap fraction of a pulp cooking process.

8. A method as claimed in any one of the preceding claims, **characterized in that** the phytosterol concentrate is in solid form.

9. A method as claimed in claim 8, **characterized in that** the phytosterol content of the phytosterol concentrate is over 80 %, preferably 90 to 98 %.

10. A method as claimed in any one of claims 1 to 7, **characterized in that** the phytosterol concentrate is in the form of a liquid or a suspension.

11. A method as claimed in claim 10, **characterized in that** the phytosterol content of the phytosterol concentrate is 20 to 80 %, preferably 40 to 60 %.

12. A method as claimed in any one of the preceding claims, **characterized in that** the method is carried out as a semi-continuous process.

13. A method as claimed in any one of preceding claims, **characterized in that** the method comprises additional steps, in which the carbon dioxide that contains impurities is separated from the phytosterol concentrate and the pure phytosterol concentrate is recovered.

14. A method as claimed in any one of claims 1 to 12, **characterized in that** the method further comprises one or more extraction and/or crystallization steps prior to the treatment with the pressurized fluid and/or thereafter.

15. A method as claimed in any one of claims 1 to 5, **characterized in that** a phytosterol preparation dissolved in a supercritical fluid is used as the starting material.

16. A purified phytosterol concentrate, **characterized by** being prepared by a method as claimed in any one of claims 1 to 13.

17. A solid phytosterol concentrate, **characterized in that** the phytosterol content therein is over 80 %, preferably 90 to 98 % and that it is substantially free of substances producing undesirable odours and flavours and of other harmful components.

18. A phytosterol concentrate in the form of a liquid or a suspension, **characterized in that** the phytosterol content therein is 20 to 80 %, preferably 40 to 60 % and that it is substantially free of substances producing undesirable odours and flavours and of other harmful components.

19. A foodstuff premix, **characterized by** containing the phytosterol concentrate as claimed in any one of claims 16 to 18 and conventional foodstuff raw material.

20. A premix as claimed in claim 19, **characterized by** containing the phytosterol concentrate as claimed in any one of claims 16 to 18 and one or more minerals selected from the group of magnesium, calcium and potassium.

21. A premix as claimed in claim 19, **characterized by** containing the phytosterol concentrate as claimed in any one of claims 16 to 18 and an emulsifier and optionally a fat component.

22. A premix as claimed in claim 19 for sweets, **characterized by** containing the phytosterol concentrate as claimed in any one of claims 16 to 18 and conventional raw material for sweets.

23. A premix as claimed in claim 19 for chocolate, **characterized by** containing the phytosterol concentrate as claimed in any one of claims 16 to 18 and conventional raw material for chocolate.

24. A premix as claimed in claim 19 for sausage products, **characterized by** containing the phytosterol concentrate as claimed in any one of claims 16 to 18 and conventional raw material for sausage products.

25. A premix as claimed in claim 19 for bakery products, **characterized by** containing the phytosterol concentrate as claimed in any one of claims 16 to 18 and conventional raw material for bakery products.

26. A premix as claimed in claim 19 for milk products, **characterized by** containing the phytosterol concentrate as claimed in any one of claims 16 to 18 and conventional raw material for milk products.

27. A foodstuff, **characterized by** containing the phytosterol concentrate as claimed in any one of claims 16 to 18 or the foodstuff premix as claimed in any one of claims 19 to 26.

28. A premix for animal feeds, **characterized by** containing the phytosterol concentrate as claimed in any one of claims 16 to 18 and conventional raw material for animal feeds.

29. An animal feed, **characterized by** containing the phytosterol concentrate as claimed in any one of claims 16 to 18 or the premix for animal feed as claimed in claim 28.

30. A premix for dietary supplement products, **characterized by** containing the phytosterol concentrate as claimed in any one of claims 16 to 18 and conventional raw material for dietary supplement products.

31. A dietary supplement product, **characterized by** containing the phytosterol concentrate as claimed in any one of claims 16 to 18 or the premix as claimed in claim 30.

32. A premix for pharmaceuticals, **characterized by** containing the phytosterol concentrate as claimed in any one of claims 16 to 18 and conventional raw material for pharmaceuticals.

33. A pharmaceutical, **characterized by** containing the phytosterol concentrate as claimed in any one of claims 16 to 18 or the premix as claimed in claim 32.

## Patentansprüche

1. Verfahren zur Reinigung von Phytosterol-Konzentraten, **dadurch gekennzeichnet, daß** das Phytosterol-Konzentrat, das einen Phytosterol-Gehalt von 20 bis 80 % oder über 80 % hat, mit einem unter Druck gesetzten Fluid in Kontakt gebracht wird.

2. Verfahren nach Anspruch 1, **dadurch gekennzeichnet, daß** das unter Druck gesetzte Fluid eine nicht-polare Substanz mit Lebensmittelqualität oder ein Gemisch solcher Substanzen ist, die/das bei normalem atmosphärischem Druck gasförmig sind/ist.

3. Verfahren nach Anspruch 2, **dadurch gekennzeichnet, daß** das unter Druck gesetzte Fluid hauptsächlich aus Kohlendioxid besteht.

4. Verfahren nach einem der Ansprüche 1 bis 3, **dadurch gekennzeichnet, daß** das Verfahren bei einer Temperatur von -20°C bis 180°C und bei einem Druck von 50 bis 600 bar durchgeführt wird.

5. Verfahren nach Anspruch 4, **dadurch gekennzeichnet, daß** das Verfahren bei einer Temperatur von 10 bis 75°C, vorzugsweise bei 20 bis 60°C, am bevorzugtesten bei 35 bis 40°C und bei einem Druck von 80 bis 300 bar, vorzugsweise bei 150 bis 250 bar durchgeführt wird.

6. Verfahren nach einem der Ansprüche 1 bis 5, **dadurch gekennzeichnet, daß** das Phytosterol-Konzentrat aus einer Destillationsfraktion einer Wasserdampfraffination von Pflanzenölen erhalten wird.

7. Verfahren nach einem der Ansprüche 1 bis 5, **dadurch gekennzeichnet, daß** das Phytosterol-Konzentrat aus einer Seifenfraktion eines Pulpe-Kochverfahrens erhalten wird.

8. Verfahren nach einem der vorangehenden Ansprüche, **dadurch gekennzeichnet, daß** das Phytosterol-Konzentrat in fester Form vorliegt.

9. Verfahren nach Anspruch 8, **dadurch gekennzeichnet, daß** der Phytosterol-Gehalt des Phytosterol-Konzentrats über 80 % liegt, vorzugsweise 90 bis 98 % ist.

10. Verfahren nach einem der Ansprüche 1 bis 7, **dadurch gekennzeichnet, daß** das Phytosterol-Konzentrat in Form einer Flüssigkeit oder einer Suspension vorliegt.

11. Verfahren nach Anspruch 10, **dadurch gekennzeichnet, daß** der Phytosterol-Gehalt des Phytosterol-Konzentrats 20 bis 80 %, vorzugsweise 40 bis 60 % ist.

12. Verfahren nach einem der vorangehenden Ansprüche, **dadurch gekennzeichnet, daß** das Verfahren als ein halbkontinuierliches Verfahren durchgeführt wird.

13. Verfahren nach einem der vorangehenden Ansprüche, **dadurch gekennzeichnet, daß** das Verfahren zusätzliche Stufen umfaßt, in denen das Kohlendioxid, das Verunreinigungen enthält, vom Phytosterol-Konzentrat abgetrennt wird und das reine Phytosterol-Konzentrat isoliert wird.

14. Verfahren nach einem der Ansprüche 1 bis 12, **dadurch gekennzeichnet, daß** das Verfahren außerdem eine oder mehrere Extraktionsstufen und/oder Kristallisationsstufen vor der Behandlung mit dem unter Druck gesetzten Fluid und/oder danach umfaßt.

15. Verfahren nach einem der Ansprüche 1 bis 5, **dadurch gekennzeichnet, daß** ein Phytosterol-Präparat, das in einem superkritischen Fluid gelöst ist, als Ausgangsmaterial verwendet wird.

16. Gereinigtes Phytosterol-Konzentrat, **dadurch gekennzeichnet, daß** es durch ein Verfahren nach einem der Ansprüche 1 bis 3 hergestellt.

17. Festes Phytosterol-Konzentrat, **dadurch gekennzeichnet, daß** der Phytosterol-Gehalt desselben über 80 % liegt, vorzugsweise 90 bis 98 % ist, und daß es im wesentlichen frei von Substanzen, die unerwünschte Gerüche und Aromastoffe produzieren, und frei von anderen gefährlichen Komponenten ist.

18. Phytosterol-Konzentrat in Form einer Flüssigkeit oder einer Suspension, **dadurch gekennzeichnet, daß** der Phytosterol-Gehalt desselben 20 bis 80 %, vorzugsweise 40 bis 60 % ist und daß es im wesentlichen frei von Substanzen, die unerwünschte Gerüche und Aromastoffe produzieren, und frei von anderen gefährlichen Komponenten ist.

19. Nahrungsmittel-Vormischung, **dadurch gekennzeichnet, daß** sie das Phytosterol-Konzentrat nach einem der Ansprüche 16 bis 18 und herkömmliches Nahrungsmittel-Rohstoffmaterial enthält.

20. Vormischung nach Anspruch 19, **dadurch gekennzeichnet, daß** sie das Phytosterol-Konzentrat nach einem der Ansprüche 16 bis 18 und ein Mineral oder mehrere Mineralien, ausgewählt aus der Gruppe bestehend aus Magnesium, Calcium und Kalium, enthält.

21. Vormischung nach Anspruch 19, **dadurch gekennzeichnet, daß** sie das Phytosterol-Konzentrat nach einem der Ansprüche 16 bis 18 und ein Emulgiermittel und gegebenenfalls eine Fettkomponente enthält.

22. Vormischung nach Anspruch 19 für Süßigkeiten, **dadurch gekennzeichnet, daß** sie das Phytosterol-Konzentrat nach einem der Ansprüche 16 bis 18 und ein herkömmliches Rohstoffmaterial für Süßigkeiten enthält.

23. Vormischung nach Anspruch 19 für Schokolade, **dadurch gekennzeichnet, daß** sie das Phytosterol-Konzentrat nach einem der Ansprüche 16 bis 18 und herkömmliches Rohstoffmaterial für Schokolade enthält.

24. Vormischung nach Anspruch 19 für Wurstprodukte, **dadurch gekennzeichnet, daß** sie das Phytosterol-Konzentrat nach einem der Ansprüche 16 bis 18 und herkömmliches Rohstoffmaterial für Wurstprodukte enthält.

25. Vormischung nach Anspruch 19 für Backwaren, **dadurch gekennzeichnet, daß** sie das Phytosterol-Konzentrat nach einem der Ansprüche 16 bis 18 und herkömmliches Rohstoffmaterial für Backwaren enthält.

26. Vormischung nach Anspruch 19 für Milchprodukte, **dadurch gekennzeichnet, daß** sie das Phytosterol-Konzentrat nach einem der Ansprüche 16 bis 18 und herkömmliches Rohstoffmaterial für Milchprodukte enthält.

27. Nahrungsmittel, **dadurch gekennzeichnet daß** es das Phytosterol-Konzentrat nach einem der Ansprüche 16 bis 18 oder die Nahrungsmittelvormischung nach einem der Ansprüche bis 19 bis 26 enthält.

28. Vormischung für Tierfutter, **dadurch gekennzeichnet, daß** sie das Phytosterol-Konzentrat nach einem der Ansprüche 16 bis 18 und herkömmliches Rohstoffmaterial für Tierfutter enthält.

29. Tierfutter, **dadurch gekennzeichnet, daß** es das Phytosterol-Konzentrat nach einem der Ansprüche 16 bis 18 oder die Vormischung für Tierfutter nach Anspruch 28 enthält.

30. Vormischung für Nahrungsergänzungsprodukte, **dadurch gekennzeichnet, daß** sie das Phytosterol-Konzentrat nach einem der Ansprüche 16 bis 18 und herkömmliches Rohstoffmaterial für Nahrungsergänzungsprodukte enthält.

31. Nahrungsergänzungsprodukt, **dadurch gekennzeichnet, daß** es das Phytosterol-Konzentrat nach einem der Ansprüche 16 bis 18 oder die Vormischung nach Anspruch 30 enthält.

32. Vormischung für Pharmazeutika, **dadurch gekennzeichnet, daß** sie das Phytosterol-Konzentrat nach einem der Ansprüche 16 bis 18 und herkömmliches Rohstoffmaterial für Pharmazeutika enthält.

33. Pharmazeutikum, **dadurch gekennzeichnet, daß** es das Phytosterol-Konzentrat nach einem der Ansprüche 16 bis 18 oder die Vormischung nach Anspruch 32 enthält.

## Revendications

1. Procédé de purification de concentrés de phytostérol, **caractérisé en ce que** l'on met un concentré de phytostérol présentant une teneur en phytostérol de 20 à 80 %, ou supérieure à 80 %, en contact avec un fluide sous pression.

2. Procédé selon la revendication 1, **caractérisé en ce que** le fluide sous pression est une substance alimentaire non polaire ou un mélange de ces substances, qui est gazeux sous pression atmosphérique normale.

3. Procédé selon la revendication 2, **caractérisé en ce que** le fluide sous pression comprend principalement du dioxyde de carbone.

4. Procédé selon l'une quelconque des revendications 1 à 3, **caractérisé en ce que** l'on effectue le procédé à une température allant de -20 °C à 180 °C, et sous une pression allant de 50 à 600 bars.

5. Procédé selon la revendication 4, **caractérisé en ce que** l'on effectue le procédé à une température allant de 10 à 75 °C, de préférence de 20 à 60 °C, mieux encore de 35 à 45 °C, et sous une pression allant de 80 à 300 bars, de préférence de 150 à 250 bars.

6. Procédé selon l'une quelconque des revendications 1 à 5, **caractérisé en ce que** l'on obtient le concentré de phytostérol à partir d'une fraction de distillation de raffinage à la vapeur d'huiles végétales.

7. Procédé selon l'une quelconque des revendications 1 à 5, **caractérisé en ce que** l'on obtient le concentré de phytostérol à partir d'une fraction de savon d'un procédé de cuisson de pâte à papier.

8. Procédé selon l'une quelconque des revendications précédentes, **caractérisé en ce que** le concentré de phytostérol est sous forme solide.

9. Procédé selon la revendication 8, **caractérisé en ce que** le concentré de phytostérol présente une teneur en phytostérol supérieure à 80 %, de préférence comprise entre 90 et 98 %.

10. Procédé selon l'une quelconque des revendications 1 à 7, **caractérisé en ce que** le concentré de phytostérol est sous forme d'un liquide ou d'une suspension.

11. Procédé selon la revendication 10, **caractérisé en ce que** le concentré de phytostérol présente une teneur en phytostérol comprise entre 20 et 80 %, de préférence entre 40 et 60 %.

12. Procédé selon l'une quelconque des revendications précédentes, **caractérisé en ce que** l'on effectue le procédé comme un procédé semi-continu.

13. Procédé selon l'une quelconque des revendications précédentes, **caractérisé en ce qu'**il comprend des étapes supplémentaires, dans lesquelles on sépare le dioxyde de carbone qui contient des impuretés, du concentré de phytostérol, et on récupère un concentré de phytostérol pur.

14. Procédé selon l'une quelconque des revendications 1 à 12, **caractérisé en ce qu'**il comprend en outre une ou plusieurs étapes d'extraction et/ou de cristallisation avant et/ou après le traitement avec le fluide sous pression.

15. Procédé selon l'une quelconque des revendications 1 à 5, **caractérisé en ce que** l'on utilise une préparation de phytostérol dissoute dans un fluide supercritique comme matériau de départ.

16. Concentré de phytostérol purifié, **caractérisé en ce qu'**il est préparé par un procédé selon l'une quelconque des revendications 1 à 13.

17. Concentré de phytostérol solide, **caractérisé en ce que** la teneur en phytostérol est supérieure à 80 %, de préférence comprise entre 90 et 98 %, et **en ce qu'**il est pratiquement exempt de substances produisant des odeurs et des goûts indésirables, et d'autres composants dangereux.

18. Concentré de phytostérol sous la forme d'un liquide ou d'une suspension, **caractérisé en ce que** la teneur en phytostérol est comprise entre 20 et 80 %, de préférence entre 40 et 60 %, et **en ce qu'**il est pratiquement exempt de substances produisant des odeurs et des goûts indésirables, et d'autres composants dangereux.

19. Prémélange alimentaire, **caractérisé en ce qu'**il contient un concentré de phytostérol selon l'une quelconque des revendications 16 à 18, et une matière brute alimentaire classique.

20. Prémélange selon la revendication 19, **caractérisé en ce qu'**il contient un concentré de phytostérol selon l'une quelconque des revendications 16 à 18, et un ou plusieurs minéraux choisis dans l'ensemble constitué par le magnésium, le calcium et le potassium.

21. Prémélange selon la revendication 19, **caractérisé en ce qu'**il contient un concentré de phytostérol selon l'une quelconque des revendications 16 à 18, et un émulsifiant, et éventuellement un composant gras.

22. Prémélange selon la revendication 19, pour des sucreries, **caractérisé en ce qu'**il contient un concentré de phytostérol selon l'une quelconque des revendications 16 à 18, et une matière brute classique pour sucreries.

23. Prémélange selon la revendication 19, pour des chocolats, **caractérisé en ce qu'**il contient un concentré de phytostérol selon l'une quelconque des revendications 16 à 18, et une matière brute classique pour chocolats.

24. Prémélange selon la revendication 19, pour des produits de type saucisses, **caractérisé en ce qu'**il contient un concentré de phytostérol selon l'une quelconque des revendications 16 à 18, et une matière brute classique pour produits de type saucisses.

25. Prémélange selon la revendication 19, pour des produits de boulangerie, **caractérisé en ce qu'**il contient un concentré de phytostérol selon l'une quelconque des revendications 16 à 18, et une matière brute classique pour produits de boulangerie.

26. Prémélange selon la revendication 19, pour des produits laitiers, **caractérisé en ce qu'**il contient un concentré de phytostérol selon l'une quelconque des revendications 16 à 18, et une matière brute classique pour produits laitiers.

27. Substance alimentaire **caractérisée en ce qu'**elle contient un concentré de phytostérol selon l'une quelconque des revendications 16 à 18, ou un prémélange alimentaire selon l'une quelconque des revendications 19 à 26.

28. Prémélange pour aliments d'animaux, **caractérisé en ce qu'**il contient un concentré de phytostérol selon l'une quelconque des revendications 16 à 18, et une matière brute classique pour aliments d'animaux.

29. Aliment d'animaux, **caractérisé en ce qu'**il contient un concentré de phytostérol selon l'une quelconque des revendications 16 à 18, ou un prémélange pour aliments d'animaux selon la revendications 28.

30. Prémélange pour des produits de complément diététique, **caractérisé en ce qu'**il contient un concentré de phytostérol selon l'une quelconque des revendications 16 à 18, et une matière brute classique pour produits de complément diététique.

31. Produit de complément diététique, **caractérisé en ce qu'**il contient un concentré de phytostérol selon l'une quelconque des revendications 16 à 18, ou un prémélange selon la revendications 30.

32. Prémélange pour des produits pharmaceutiques, **caractérisé en ce qu'**il contient un concentré de phytostérol selon l'une quelconque des revendications 16 à 18, et une matière brute classique pour produits pharmaceutiques.

33. Produit pharmaceutique, **caractérisé en ce qu'**il contient un concentré de phytostérol selon l'une quelconque des revendications 16 à 18, ou un prémélange selon la revendications 32.
